# EUROPEAN PATENT APPLICATION

(11) **EP 4 692 304 A1**
(43) Date of publication of application: **11.02.2026**
(21) Application number: 24778821.9
(22) Date of filing: 16.02.2024
(51) Int. Cl.: C12M 3/00, C12M 1/00

(54) **CELL CULTURE DEVICE AND CELL CULTURE KIT**

(30) Priority: 29.03.2023 JP 2023054412
(71) Applicant: Toyo Seikan Group Holdings, Ltd., Shinagawa-ku Tokyo 141-8627 (JP)
(72) Inventor: AOYA, Masaki, Yokohama-shi, Kanagawa 240-0062 (JP); SUENAGA, Ryo, Yokohama-shi, Kanagawa 240-0062 (JP)
(74) Representative: Piticco, Lorena
(86) International application number: PCT/JP2024/005567
(87) International publication number: WO 2024/202676

(57) **Abstract**

A cell culture jig holds a cell culture bag. The cell culture jig includes a mounting platform on which the cell culture bag is mounted and a pressing member configured to press the cell culture bag mounted on the mounting platform. A slipping surface configured to reduce a frictional force acting on a contact surface between the cell culture bag mounted on the mounting platform and a pressing surface of the pressing member configured to press the cell culture bag.

## Description

### Technical Field

The present invention relates to a cell culture jig and a cell culture kit.

### Background Art

In recent years, in the fields of pharmaceutical production, gene therapy, regenerative medicine, immunotherapy, and the like, there has been a demand for efficiently culturing and inducing differentiation of cells (including tissues, microorganisms, viruses, and the like) in large quantities under artificial environments. In such cell culturing, a closed cell culture bag may be used to avoid a risk of contamination. A cell culture bag is composed of, for example, a bag main body made of a plastic film having a sealed periphery portion, and a port attached to the bag main body.

The bag main body of such a cell culture bag is made of a flexible material, and thus readily deforms and has an unstable shape. When the bag main body deforms, liquid contents flow, which can result in spheroids (clusters) of cells breaking apart or causing damage to the cells, hindering cultivation.

In response, Patent Document 1 describes a culture tray in which a cell culture bag is interposed and pressed between a bottom surface of a tray and a pressing plate in order to maintain a stable state inside the cell culture bag. Such a configuration suppresses the flow of the liquid contents caused by the deformation of the cell culture bag.

### Citation List

### Patent Literature

Patent Document 1: JP 2006-325437 A

### Summary of Invention

### Technical Problem

When the cell culture bag is interposed and pressed between two members as described above, wrinkles may form on a surface of the bag main body of the cell culture bag. Wrinkles on a bag surface cause a liquid thickness of the culture medium to be non-uniform, and may adversely affect the cell culture, and are not thus preferred.

In view of the above, the present inventors have conducted extensive studies on a cell culture jig and a cell culture kit capable of readily removing wrinkles formed on a surface of a cell culture bag in holding the cell culture bag in the cell culture jig and conducting cell culturing and, as a result, have successfully completed the present invention.

### Solution to Problem

A cell culture jig according to the present invention is a cell culture jig that holds a cell culture bag and includes a mounting platform on which the cell culture bag is mounted and a pressing member configured to press the cell culture bag mounted on the mounting platform. A slipping surface configured to reduce a frictional force acting on a contact surface between the cell culture bag mounted on the mounting platform and a pressing surface of the pressing member configured to press the cell culture bag is provided on a side of the pressing member.

A cell culture kit according to the present invention includes a cell culture bag and a cell culture jig that holds the cell culture bag, the cell culture jig including a mounting platform on which the cell culture bag is mounted and a pressing member configured to press the cell culture bag mounted on the mounting platform. A slipping surface configured to reduce a frictional force acting on a contact surface between the cell culture bag mounted on the mounting platform and a pressing surface of the pressing member pressing the cell culture bag is provided on one or both of a side of the cell culture bag and a side of the pressing member.

### Advantageous Effects of Invention

The present invention can readily remove wrinkles formed on a surface of a cell culture bag in holding the cell culture bag in a cell culture jig and conducting cell culturing.

### Brief Description of Drawings

FIG. 1 is a perspective view illustrating an outline of a cell culture jig according to an example of the present embodiment.
FIG. 2 is an explanatory view illustrating an outline of the cell culture jig according to the example of the present embodiment.
FIG. 3 is an explanatory view illustrating an outline of the cell culture jig according to another example of the present embodiment.
FIG. 4 is a perspective view illustrating an outline of a cell culture kit according to an example of the present embodiment.
FIG. 5 is a view illustrating a state in which wrinkles are formed in a main surface of a cell culture bag held by the cell culture jig.

### Description of Embodiments

Preferred embodiments of the present invention will be described below with reference to the accompanying drawings.

### First Embodiment

First, a first embodiment of the present invention will be described.

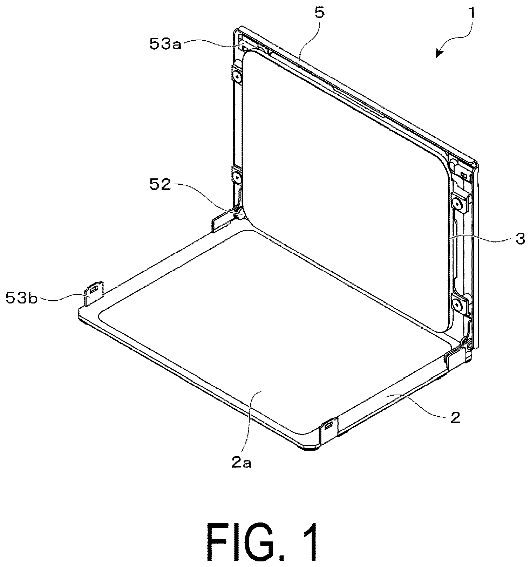

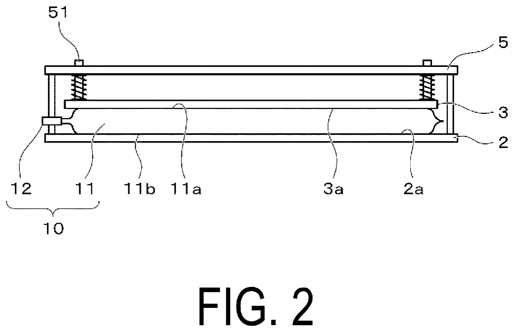

FIG. 1 is a perspective view illustrating an outline of a cell culture jig 1 according to the present embodiment, and illustrates a state in which a lid body 5 described in detail below is opened. FIG. 2 is an explanatory view illustrating an outline of the cell culture jig 1 according to the present embodiment, and schematically illustrates a state in which the cell culture jig 1 holding a cell culture bag 10 in an interior thereof is viewed from the side. In FIG. 2, to illustrate a relationship between the cell culture bag 10, a mounting platform 2, a pressing member 3, the lid body 5, and a support mechanism 51 described below, other members are omitted as appropriate.

The cell culture jig 1 of the present embodiment is used to hold the cell culture bag 10 supplied with cells to be cultured (cultured cells) and a culture medium in the interior thereof and conduct cell culturing.

Prior to description of the cell culture jig 1 of the present embodiment, the cell culture bag 10 used in the present embodiment will be briefly described.

The cell culture bag 10 used in the present embodiment includes a bag main body 11 and a port 12 attached to this bag main body 11. A planar shape of the bag main body 11 of the cell culture bag 10 may be any of various shapes, such as a polygonal shape such as a substantially rectangular shape, a square shape, or a hexagonal shape, an elliptical shape, or a circular shape. A size of the bag main body 11 is not particularly limited, but may be set to, for example, from 50 to 500 mm in length and from 50 to 500 mm in width.

The bag main body 11 can be formed by overlapping plastic films and heat-sealing peripheral portions thereof, and includes an upper surface 11a and a lower surface 11b.

The bag main body 11 is preferably formed of a plastic film having gas permeability. As for the gas permeability of the plastic film, an oxygen permeability measured at a test temperature of 37°C in accordance with the gas permeability test method of JIS K 7126 is preferably 5000 mL/(m² · day · atm) or greater.

Examples of the material used for the plastic film forming the bag main body 11 include a thermoplastic resin such as polyethylene, polypropylene, ethylene-vinyl acetate copolymer, polyester, silicone-based elastomer, polystyrene-based elastomer, and tetrafluoroethylene-hexafluoropropylene copolymer (FEP). These may be used in a single layer or by layering the same or different materials, but preferably include a layer functioning as a sealant layer in consideration of thermal fusion properties when the peripheral portions are heat-sealed.

The port 12 is formed of a tubular member through which a culture medium, cells, and the like can flow, and can be formed of, for example, a thermoplastic resin such as polyethylene, polypropylene, vinyl chloride, a polystyrene elastomer, or FEP.

Such a cell culture bag 10 is held in the interior of the cell culture jig 1, interposed between two members, and pressurized, making it possible to suppress the flow of the liquid contents inside the cell culture bag 10.

The cell culture jig 1 of the present embodiment includes the mounting platform 2 on which the cell culture bag 10 is mounted, and the pressing member 3 that presses the cell culture bag 10 mounted on the mounting platform 2 from above.

The mounting platform 2 is a plate-shaped member having a substantially rectangular planar shape larger than that of an area of the lower surface 11b of the cell culture bag 10, and includes a mounting surface 2a on which the cell culture bag 10 is horizontally mounted. When the cell culture bag 10 is mounted on the mounting platform 2, the lower surface 11b of the cell culture bag 10 comes into contact with this mounting surface 2a. A shape of the mounting surface 2a illustrated in the drawing is flat, but may be changed as appropriate in accordance with a shape of the lower surface 11b of the cell culture bag 10 as long as the cell culture bag 10 can be mounted horizontally, and is not particularly limited.

The mounting platform 2 may be partially or entirely transparent so that the state of an interior of the cell culture bag 10 and the like can be checked. In this case, the part can be formed of, for example, a synthetic resin having high transparency, such as polycarbonate, or glass.

The pressing member 3 is a plate-shaped member having a substantially rectangular planar shape corresponding to an area of the upper surface 11a of the cell culture bag 10, and includes a pressing surface 3a that is flat and substantially parallel to the mounting surface 2a of the mounting platform 2. This pressing surface 3a comes into contact with the upper surface 11a of the cell culture bag 10 mounted on the mounting platform 2 and presses the cell culture bag 10 from above.

Preferably, the pressing member 3 is partially or entirely transparent or translucent so that the state of the interior of the cell culture bag 10 and the like can be visually confirmed. For example, in consideration of opening and closing the lid body 5 described below, this part can be made of lightweight and highly transparent polycarbonate.

In the illustrated example, the pressing member 3 is supported by the lid body 5 by the support mechanism 51.

The lid body 5, as a structure, is openably and closably attached to the mounting platform 2 by a hinge 52 or the like, can open approximately 90° relative to the mounting platform 2, and can be closed to become substantially parallel with the mounting platform 2 and be fixed by latches 53a, 53b or the like. The lid body 5 is preferably configured such that a center portion of the lid body 5 is widely open and part of the pressing member 3 is exposed from the opening so that the state of the interior of the cell culture bag 10 or the like can be checked, or part of the lid body 5 is transparent or the like so that the state of the interior of the cell culture bag 10 can be visually confirmed, but the form of the lid body 5 is not particularly limited.

The support mechanism 51 is constituted by guide pins extending from the four corners of the pressing member 3 toward the lid body 5 and penetrating a frame of the lid body 5, and urging means for urging the pressing member 3 in a direction away from the lid body 5, and supports the pressing member 3 in a state of being substantially parallel to the lid body 5. Although a spring member is provided between the frame of the lid body 5 and the pressing member 3 as the urging means herein, a repelling force of a magnet, for example, may be utilized as the urging means instead of the spring member. In this way, preferably the pressing member 3 is provided so that a distance to the lid body 5 can be changed while maintaining a substantially parallel state with the lid body 5, and the pressing member 3 is urged in a direction away from the lid body 5 (that is, in a direction in which the pressing member 3 approaches the mounting platform 2 in a state in which the lid body 5 is closed) by utilizing a repulsive force of the spring member or the repelling force of the magnet. According to such a mode, after the cell culture bag 10 is mounted on the mounting platform 2 and the lid body 5 is closed, even if variation exists in a thickness of the cell culture bag 10 due to inflow and outflow of the culture medium, for example, it is possible to maintain a state in which the cell culture bag 10 is sandwiched and pressed between the mounting platform 2 and the pressing member 3 while the pressing member 3 varies the distance to the mounting platform 2 in accordance with the thickness of the cell culture bag 10. Preferably, the support mechanism 51 supports the pressing member 3 so that the distance to the lid body 5 can be changed, and includes the urging means in order to remove wrinkles on a surface of the cell culture bag 10 by a sheet member 4, which will be described in detail below.

When the cell culture bag 10 mounted on the mounting platform 2 is pressed by such a pressing member 3, the bag main body 11 of the cell culture bag 10 may bend, forming wrinkles in the upper surface 11a. This tends to occur more readily as the cell culture bag 10 increases in size. The wrinkles, once formed, cannot be easily removed due to a frictional force acting on a contact surface between a surface of the bag main body 11 of the cell culture bag 10 and the pressing member 3. In a state in which the surface of the cell culture bag 10 is wrinkled, a liquid thickness of the culture medium filled in the interior becomes uneven. Further, at the time of culture medium exchange, a flow rate of the culture medium may locally increase, causing the cells to readily flow. Thus, a state in which wrinkles exist on the surface of the bag main body 11 of the cell culture bag 10 may adversely affect cell cultivation, and thus is not preferred.

In response, the cell culture jig 1 in the present embodiment includes a slipping surface on the pressing member 3 side. More specifically, the pressing surface 3a of the pressing member 3 that comes into contact with the upper surface 11a of the cell culture bag 10 is a slipping surface. This reduces the frictional force acting on the contact surface between the cell culture bag 10 mounted on the mounting platform 2 and the pressing member 3 that presses the cell culture bag 10.

The pressing surface 3a of the pressing member 3 can be formed as a slipping surface by, for example, applying a roughening treatment such as blasting to form a roughened surface having surface roughness (Ra) of from 0.01 to 0.70 µm. The degree of surface roughness can be changed as appropriate in accordance with the configuration of the materials forming the pressing member 3 and the bag main body 11 of the cell culture bag 10, and the like. For example, when the pressing surface 3a of the pressing member 3 is configured such that a coefficient of kinetic friction between the pressing surface 3a of the pressing member 3 and the upper surface 11a of the cell culture bag 10 is greater than 0 and 1.0 or less, preferably from 0.05 to 1.0, more preferably from 0.1 to 0.7, and even more preferably from 0.3 to 0.7, it is possible to reduce a number of oscillations of the pressing member 3 needed to remove wrinkles from the surface of the cell culture bag 10, which is preferable.

According to such a cell culture jig 1, when the cell culture bag 10 is mounted on the mounting platform 2, the lid body 5 is closed, and the cell culture bag 10 is pressed by the pressing member 3, wrinkles can be readily removed as follows, even if the wrinkles are formed in the upper surface 11a of the cell culture bag 10.

When wrinkles on the surface of the bag main body 11 are confirmed, the pressing member 3 is oscillated in a direction away from the surface of the bag main body 11 of the cell culture bag 10. Then, the upper surface 11a of the bag main body 11 moves as the contents such as the culture medium in the cell culture bag 10 slightly shake, the pressing surface 3a of the pressing member 3 functions as a slipping surface having slipperiness, the upper surface 11a of the cell culture bag 10 slides on the pressing surface 3a having a flat, plate shape, and the upper surface 11a is flattened along the shape of the pressing surface 3a. In this way, the wrinkles on the surface of the bag main body 11 of the cell culture bag 10 can be removed.

To oscillate the pressing member 3 in a direction away from the surface of the bag main body 11, the lid body 5 supporting the pressing member 3 may be moved. Alternatively, in a case in which the pressing member 3 is supported by the support mechanism 51 described above so that the distance to the lid body 5 can be changed and is urged away from the lid body 5 by the urging means, it is possible perform oscillation by pressing the pressing member 3 upward toward the lid body 5 or the like with fingers or the like placed on an edge side of the pressing member 3, even after the lid body 5 is locked to the mounting platform 2 by the latches 53a, 53b, or the like.

### Second Embodiment

Next, a second embodiment of the present invention will be described.

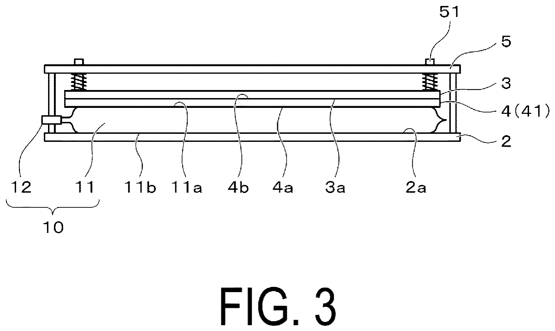

FIG. 3 is an explanatory view illustrating an outline of the cell culture jig 1 according to the present embodiment, and schematically illustrates a state in which the cell culture jig 1 holding the cell culture bag 10 in the interior thereof is viewed from the side. In FIG. 3, as in FIG. 2, to illustrate the relationship between the cell culture bag 10, the mounting platform 2, the pressing member 3, the sheet member 4, the lid body 5, and the support mechanism 51 described below, other members are omitted as appropriate.

In the first embodiment described above, the pressing surface 3a of the pressing member 3 that comes into contact with the upper surface 11a of the cell culture bag 10 is used as a slipping surface, and the slipping surface that reduces the frictional force acting on the contact surface between the cell culture bag 10 mounted on the mounting platform 2 and the pressing member 3 that presses the cell culture bag 10 is provided on the pressing member 3 side. In contrast, in the present embodiment, a configuration is adopted in which the pressing surface 3a of the pressing member 3 is not formed as a slipping surface. Rather, the sheet member 4 including a slipping surface is interposed between the cell culture bag 10 and the pressing member 3, and the pressing member 3 side is provided with the slipping surface that reduces the frictional force acting on the contact surface between the cell culture bag 10 mounted on the mounting platform 2 and the pressing member 3 that presses the cell culture bag 10.

The sheet member 4 is a flexible sheet that can be adhered to the pressing surface 3a of the pressing member 3, and is preferably transparent or translucent so that the state of the interior of the cell culture bag 10 and the like can be visually recognized. The sheet member 4 can be formed from thermoplastic resin. However, as long as a bag contact surface 4a can be made into a slipping surface as described below, the material used for the sheet member 4 is not particularly limited.

The sheet member 4 in the present embodiment includes the bag contact surface 4a that comes into contact with the upper surface 11a of the cell culture bag 10 on a side opposite to a surface facing the pressing surface 3a of the pressing member 3. In the present embodiment, this bag contact surface 4a serves as the slipping surface.

The bag contact surface 4a of the sheet member 4 can be formed into the slipping surface by, for example, undergoing a roughening treatment such as blasting to form a roughened surface having surface roughness (Ra) of from 0.01 to 0.70 µm.

Alternatively, the sheet member 4 may be formed by using a material having high self-lubricating properties so that the surface thereof serves as the slipping surface. Examples of such a material include polytetrafluoroethylene, polyacetal, ultra-high molecular weight polyethylene, and monomer cast (MC) nylon.

Alternatively, the bag contact surface 4a of the sheet member 4 may be formed so as to include an outer layer imparted with slipperiness. In this case, the outer layer of the sheet member 4 may contain a slip agent such as silicone, a surfactant, petroleum wax, synthetic paraffin, liquid paraffin, fatty acid ester, or an amide compound. Even materials that cannot be used to construct the cell culture bag 10 due to the risk of affecting the cultured cells can be used for the sheet member 4 without issue. Therefore, various materials can be used for the sheet member 4 to impart desired physical properties.

Thus, when the bag contact surface 4a of the sheet member 4 is configured such that a coefficient of kinetic friction between the bag contact surface 4a of the sheet member 4 and the upper surface 11a of the cell culture bag 10 is greater than 0 and 1.0 or less, preferably from 0.05 to 1.0, more preferably from 0.1 to 0.7, and even more preferably from 0.3 to 0.7, the number of oscillations of the pressing member 3 needed to remove wrinkles from the surface of the cell culture bag 10 can be reduced, which is preferable.

According to the cell culture jig 1 of the present embodiment, when the cell culture bag 10 is mounted on the mounting platform 2, the lid body 5 is closed, and the cell culture bag 10 is pressed by the pressing member 3, wrinkles in the surface of the bag main body 11 of the cell culture bag 10 can be readily removed, even if the wrinkles are formed in the upper surface 11a of the cell culture bag 10. That is, when wrinkles are confirmed, the pressing member 3 is oscillated in a direction away from the surface of the bag main body 11 of the cell culture bag 10 in the same manner as in the first embodiment. Then, the bag contact surface 4a of the sheet member 4 adhered to the pressing surface 3a of the pressing member 3 functions as a slipping surface having slipperiness, the upper surface 11a side of the cell culture bag 10 slides on the pressing surface 3a having a flat plate shape, and the surface of the bag main body 11 is flattened along the shape of the pressing surface 3a, making it possible to remove the wrinkles in the surface of the bag main body 11 of the cell culture bag 10.

Although the present embodiment differs from the first embodiment in the above-described respects, the other configurations are similar to those of the first embodiment, and thus redundant descriptions will be omitted.

Examples of the cell culture jig 1 including, on the pressing member 3 side, a slipping surface that reduces the frictional force acting on the contact surface between the cell culture bag 10 mounted on the mounting platform 2 and the pressing surface 3a of the pressing member 3 that presses the cell culture bag 10 have been described above. However, the present invention is not limited to the above-described embodiments. Such a slipping surface may be provided on the cell culture bag 10 side, and an example thereof will be described below.

### Third Embodiment

A third embodiment of the present invention will now be described.

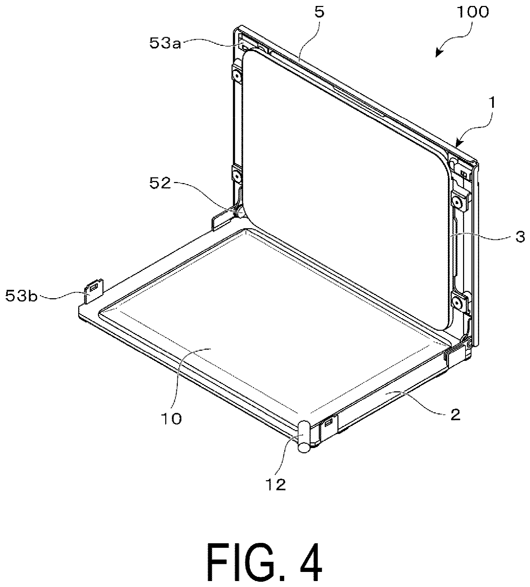

FIG. 4 is a perspective view illustrating an outline of a cell culture kit 100 according to the present embodiment, and illustrates a state in which the lid body 5 is opened. The cell culture kit 100 includes the cell culture bag 10, and the cell culture jig 1 including the mounting platform 2 on which the cell culture bag is mounted and the pressing member 3 that presses the cell culture bag 10 mounted on the mounting platform 2.

In the first embodiment described above, the pressing surface 3a of the pressing member 3 that comes into contact with the upper surface 11a of the cell culture bag 10 is used as the slipping surface, and the slipping surface that reduces the frictional force acting on the contact surface between the cell culture bag 10 mounted on the mounting platform 2 and the pressing member 3 that presses the cell culture bag 10 is provided on the pressing member 3 side. In contrast, in the present embodiment, the pressing surface 3a of the pressing member 3 is not used as the slipping surface, but the upper surface 11a of the cell culture bag 10 mounted on the cell culture jig 1 is used as the slipping surface, and the slipping surface that reduces the frictional force acting on the contact surface between the cell culture bag 10 mounted on the mounting platform 2 and the pressing member 3 pressing the cell culture bag 10 is provided on the cell culture bag 10 side.

The upper surface 11a of the cell culture bag 10 included in the cell culture kit of the present embodiment can be formed of, for example, a thermoplastic resin such as polyethylene, polypropylene, ethylene-vinyl acetate copolymer, polyester, silicone-based elastomer, polystyrene-based elastomer, and tetrafluoroethylene-hexafluoropropylene copolymer (FEP).

The upper surface 11a of the cell culture bag 10 can be formed into a slipping surface by, for example, undergoing a roughening treatment such as blasting to form a roughened surface on the pressing member 3 having surface roughness (Ra) of from 0.01 to 0.70 µm. The degree of surface roughness can be changed as appropriate in accordance with the configuration of the materials forming the pressing member 3 and the bag main body 11 of the cell culture bag 10, and the like.

Alternatively, the upper surface 11a of the cell culture bag 10 may be formed by using a material having high self-lubricating properties so that the surface thereof serves as a slipping surface. Examples of such a material include polytetrafluoroethylene and ultra-high molecular weight polyethylene.

Thus, when the upper surface 11a of the cell culture bag 10 is configured such that a coefficient of kinetic friction between the pressing surface 3a of the pressing member 3 and the upper surface 11a of the cell culture bag 10 is greater than 0 and 1.0 or less, preferably from 0.05 to 1.0, more preferably from 0.1 to 0.7, and even more preferably from 0.3 to 0.7, the number of oscillations of the pressing member 3 needed to remove wrinkles from the surface of the cell culture bag 10 can be reduced, which is preferable.

According to such a cell culture kit 100, when the cell culture bag 10 is mounted on the mounting platform 2, the lid body 5 is closed, and the cell culture bag 10 is pressed by the pressing member 3, wrinkles in the surface of the bag main body 11 of the cell culture bag 10 can be readily removed, even if the wrinkles are formed in the upper surface 11a of the cell culture bag 10. That is, when wrinkles are confirmed, the pressing member 3 is oscillated so as to separate away from the surface of the bag main body 11 of the cell culture bag 10 in the same manner as in the first embodiment. Then, the upper surface 11a of the cell culture bag 10 functions as a slipping surface having slipperiness, the upper surface 11a of the cell culture bag 10 slides on the pressing surface 3a having a flat plate shape, and the surface of the bag main body 11 is flattened along the shape of the pressing surface 3a, making it possible to remove the wrinkles in the surface of the bag main body 11 of the cell culture bag 10.

Although the present embodiment differs from the first embodiment in the above-described respects, the other configurations are similar to those of the first embodiment, and thus redundant descriptions will be omitted.

### Fourth Embodiment

Next, a fourth embodiment of the present invention will be described.

The second embodiment described above has a configuration in which the sheet member 4 is interposed between the cell culture bag 10 and the pressing member 3, the sheet member 4 is adhered to the pressing surface 3a of the pressing member 3 so that the bag contact surface 4a of the sheet member 4 coming into contact with the upper surface 11a of the cell culture bag 10 serves as the slipping surface and in which the slipping surface that reduces the frictional force acting on the contact surface between the cell culture bag 10 mounted on the mounting platform 2 and the pressing surface 3a of the pressing member 3 is provided on the pressing member side. In contrast, the present embodiment has a configuration in which the sheet member 4 is adhered to the upper surface 11a of the cell culture bag 10, a pressing member facing surface 4b of the sheet member 4 facing the pressing surface 3a of the pressing member 3 is used as the slipping surface and in which the slipping surface that reduces the friction force acting on the contact surface between the cell culture bag 10 mounted on the mounting platform 2 and the pressing surface 3a of the pressing member 3 is provided on the cell culture bag 10 side (see FIG. 3).

According to such a cell culture kit 100, when the cell culture bag 10 is mounted on the mounting platform 2, the lid body 5 is closed, and the cell culture bag 10 is pressed by the pressing member 3, wrinkles in the surface of the bag main body 11 of the cell culture bag 10 can be readily removed, even if the wrinkles are formed in the upper surface 11a of the cell culture bag 10. That is, when wrinkles are confirmed, the pressing member 3 is oscillated in a direction away from the surface of the bag main body 11 of the cell culture bag 10 in the same manner as in the first embodiment. Then, the pressing member facing surface 4b of the sheet member 4 adhered to the upper surface 11a of the cell culture bag 10 functions as a slipping surface having slipperiness, the upper surface 11a side of the cell culture bag 10 slides on the pressing surface 3a having a flat plate shape, and the surface of the bag main body 11 is flattened along the shape of the pressing surface 3a, making it possible to remove the wrinkles in the surface of the bag main body 11 of the cell culture bag 10.

Although the present embodiment differs from the second embodiment in the above-described respects, the other configurations are similar to those of the second embodiment, and thus redundant descriptions will be omitted.

Thus, providing the slipping surface that reduces the friction force acting on the contact surface between the cell culture bag 10 mounted on the mounting platform 2 and the pressing surface 3a of the pressing member 3 that presses the cell culture bag 10 on either the cell culture bag 10 side or the pressing member 3 side can readily remove wrinkles formed in the surface of the bag main body 11 of the cell culture bag 10 when the cell culture bag 10 is held in the interior of the cell culture jig 1. Needless to say, the slipping surface that reduces the friction force acting on the contact surface between the cell culture bag 10 mounted on the mounting platform 2 and the pressing surface 3a of the pressing member 3 that presses the cell culture bag 10 is provided on one or both of a side of the cell culture bag 10 and a side of the pressing member 3.

### Examples

The present invention will now be described in greater detail with reference to specific examples.

### Example 1

The pressing surface 3a of the pressing member 3 formed of polycarbonate underwent blasting using a shot material (manufactured by Fuji Manufacturing Co., Ltd.) in which glass abrasive grains having an average particle size of 11 µm were kneaded into a rubber core having an average particle size of 350 µm, bringing the surface roughness (Ra) to 0.118 µm. The transparency (haze) of the pressing member 3 was 36.22. The cell culture bag 10 used was a bag in which the bag main body 11 was molded from polyethylene and the planar shape of the bag main body 11 was 26.7 cm in length by 36.4 cm in width. The coefficient of kinetic friction between the pressing surface 3a of the pressing member 3 and the upper surface 11a of the cell culture bag 10 was 0.39.

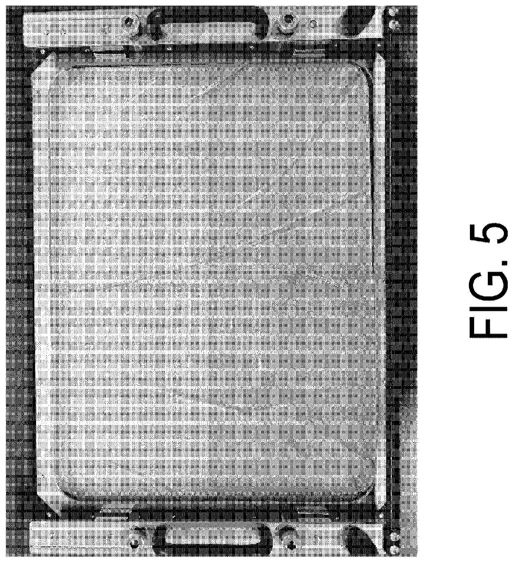

When the cell culture bag 10 filled with about 400 ml of the culture medium was mounted on onto the cell culture jig 1 illustrated in FIG. 1 and provided with the pressing member 3 having the configuration described above, and the lid body 5 was closed and locked by the latches, wrinkles were formed in the surface of the bag main body 11 of the cell culture bag 10. FIG. 5 is a view illustrating a state in which wrinkles are formed in the surface of the bag main body 11 of the cell culture bag 10 held in the interior of the cell culture jig 1 and pressed by the pressing member 3. Subsequently, when fingers were placed on the edge side of the pressing member 3 and the pressing member 3 was oscillated once so as to approach the lid body 5, the removal of the wrinkles was confirmed.

### Example 2

The cell culture jig 1 and the cell culture bag 10 were prepared in the same manner as in example 1, except that the blasting was performed using a shot material (manufactured by Fuji Manufacturing Co., Ltd.) in which glass abrasive grains having an average particle size of 0.5 µm were kneaded into a rubber core having an average particle size of 350 µm, and the pressing surface 3a of the pressing member 3 had surface roughness (Ra) of 0.018 µm. The transparency (haze) of the pressing member 3 was 4.58, and the coefficient of kinetic friction between the pressing surface 3a of the pressing member 3 and the upper surface 11a of the cell culture bag 10 was 0.61.

When the cell culture bag 10 was held in the interior of the cell culture jig 1 of example 2 in the same manner as in example 1, wrinkles were formed in the surface of the bag main body 11 of the cell culture bag 10. Subsequently, when fingers were placed on the edge of the pressing member 3 and the pressing member 3 was oscillated twice so as to approach the lid body 5, the removal of the wrinkles was confirmed.

### Example 3

The cell culture jig 1 and the cell culture bag 10 were prepared in the same manner as in example 1, except that the blasting was performed using a shot material (manufactured by Fuji Manufacturing Co., Ltd.) in which glass abrasive grains having an average particle size of 1.2 µm were kneaded into a rubber core having an average particle size of 350 µm, and the pressing surface 3a of the pressing member 3 had surface roughness (Ra) of 0.037 µm. The transparency (haze) of the pressing member 3 was 7.76, and the coefficient of kinetic friction between the pressing surface 3a of the pressing member 3 and the upper surface 11a of the cell culture bag 10 was 0.55.

When the cell culture bag 10 was held in the interior of the cell culture jig 1 of example 3 in the same manner as in example 1, wrinkles were formed in the surface of the bag main body 11 of the cell culture bag 10. Subsequently, when fingers were placed on the edge of the pressing member 3 and the pressing member 3 was oscillated twice so as to approach the lid body 5, the removal of the wrinkles was confirmed.

### Example 4

The cell culture jig 1 and the cell culture bag 10 were prepared in the same manner as in example 1, except that the blasting was performed using a shot material (manufactured by Fuji Manufacturing Co., Ltd.) in which glass abrasive grains having an average particle size of 4 µm were kneaded into a rubber core having an average particle size of 350 µm, and the pressing surface 3a of the pressing member 3 had surface roughness (Ra) of 0.062 µm. The transparency (haze) of the pressing member 3 was 17.42, and the coefficient of kinetic friction between the pressing surface 3a of the pressing member 3 and the upper surface 11a of the cell culture bag 10 was 0.41.

When the cell culture bag 10 was held in the interior of the cell culture jig 1 of example 4 in the same manner as in example 1, wrinkles were formed in the surface of the bag main body 11 of the cell culture bag 10. Subsequently, when fingers were placed on the edge of the pressing member 3 and the pressing member 3 was oscillated seven times so as to approach the lid body 5, the removal of the wrinkles was confirmed.

### Example 5

The cell culture jig 1 and the cell culture bag 10 were prepared in the same manner as in example 1, except that the blasting was performed using a shot material (manufactured by Fuji Manufacturing Co., Ltd.) in which glass abrasive grains having an average particle size of 20 µm were kneaded into a rubber core having an average particle size of 350 µm, and the pressing surface 3a of the pressing member 3 had surface roughness (Ra) of 0.254 µm. The transparency (haze) of the pressing member 3 was 74.4, and the coefficient of kinetic friction between the pressing surface 3a of the pressing member 3 and the upper surface 11a of the cell culture bag 10 was 0.44.

When the cell culture bag 10 was held in the interior of the cell culture jig 1 of example 5 in the same manner as in example 1, wrinkles were formed in the surface of the bag main body 11 of the cell culture bag 10. Subsequently, when fingers were placed on the edge of the pressing member 3 and the pressing member 3 was oscillated eight times so as to approach the lid body 5, the removal of the wrinkles was confirmed.

### Example 6

The cell culture jig 1 and the cell culture bag 10 were prepared in the same manner as in example 1, except that the blasting was performed using a shot material (manufactured by Fuji Manufacturing Co., Ltd.) in which glass abrasive grains having an average particle size of 40 µm were kneaded into a rubber core having an average particle size of 350 µm, and the pressing surface 3a of the pressing member 3 had surface roughness (Ra) of 0.658 µm. The transparency (haze) of the pressing member 3 was 83.26, and the coefficient of kinetic friction between the pressing surface 3a of the pressing member 3 and the upper surface 11a of the cell culture bag 10 was 0.43.

When the cell culture bag 10 was held in the interior of the cell culture jig 1 of example 6 in the same manner as in example 1, wrinkles were formed in the surface of the bag main body 11 of the cell culture bag 10. Subsequently, when fingers were placed on the edge of the pressing member 3 and the pressing member 3 was oscillated 12 times so as to approach the lid body 5, the removal of the wrinkles was confirmed.

### Comparative Example

The cell culture jig 1 and the cell culture bag 10 were prepared in the same manner as in example 1 except that the blasting was not performed on the pressing surface 3a of the pressing member 3. The surface roughness (Ra) of the pressing surface 3a of the pressing member 3 was 0.004 µm, the transparency (haze) of the pressing member 3 was 0.72, and the coefficient of kinetic friction between the pressing surface 3a of the pressing member 3 and the upper surface 11a of the cell culture bag 10 was 2.02.

When the cell culture bag 10 was held in the interior of the cell culture jig 1 of the comparative example in the same manner as in example 1, wrinkles were formed in the surface of the bag main body 11 of the cell culture bag 10. Subsequently, fingers were placed on the edge of the pressing member 3 and the pressing member 3 was oscillated 30 times so as to approach the lid body 5, but the wrinkles were not removed.

Although the present invention has been described above with reference to the preferred embodiments, the present invention is not limited only to the embodiments described above, and various modifications can be made within the scope of the present invention.

For example, although a configuration is adopted in which the friction force between the upper surface 11a of the cell culture bag 10 and the pressing member 3 is reduced in the present embodiment, the slipping surface that reduces the friction force acting on the contact surface between the cell culture bag 10 and the mounting platform 2 may be provided on the mounting platform 2 side or the cell culture bag 10 side so as to remove wrinkles formed in the lower surface 11b of the cell culture bag 10. The mounting surface 2a may be used as the slipping surface, or the lower surface 11b of the cell culture bag 10 may be used as the slipping surface. The sheet member 4 including the slipping surface may be interposed between the cell culture bag 10 and the mounting platform 2. In this case, to remove the wrinkles formed in the lower surface 11b of the cell culture bag 10, the bag main body 11 of the cell culture bag 10 need only be oscillated by being lifted with fingers.

In the illustrated examples, the shape of the lower surface 11b of the bag main body 11 is flat similarly to the upper surface 11a, but a plurality of recess portions, each serving as a cell culture part, may be formed in the lower surface 11b. In this case, openings or recess portions may be formed in the mounting surface 2a of the mounting platform 2 in shapes that receive each of the plurality of recess portions formed in the lower surface 11b of the cell culture bag 10, and the recess portions in the lower surface 11b may be supported in a non-contact manner so as to avoid collapse or deformation of the recess portions and prevent the outflow of cells from inside the recess portions.

### Reference Signs List

1 Cell culture jig
2 Mounting platform
3 Pressing member
4 Sheet member
5 Lid body
10 Cell culture bag

## Claims

1. A cell culture jig that holds a cell culture bag, the cell culture jig comprising:
a mounting platform on which the cell culture bag is mounted; and
a pressing member configured to press the cell culture bag mounted on the mounting platform, wherein
a slipping surface configured to reduce a frictional force acting on a contact surface between the cell culture bag mounted on the mounting platform and a pressing surface of the pressing member configured to press the cell culture bag is provided on a side of the pressing member.

2. The cell culture jig according to claim 1, wherein a sheet member including the slipping surface is interposed between the cell culture bag and the pressing member.

3. The cell culture jig according to claim 1 or 2, wherein the slipping surface is a roughened surface obtained by roughening treatment.

4. The cell culture jig according to claim 3, wherein the roughened surface has surface roughness (Ra) of from 0.01 to 0.70 µm.

5. The cell culture jig according to claim 2, wherein the sheet member is made of a self-lubricating material.

6. A cell culture kit, comprising:
a cell culture bag; and
a cell culture jig that holds the cell culture bag, the cell culture jig comprising a mounting platform on which the cell culture bag is mounted and a pressing member configured to press the cell culture bag mounted on the mounting platform, wherein
a slipping surface configured to reduce a frictional force acting on a contact surface between the cell culture bag mounted on the mounting platform and a pressing surface of the pressing member pressing the cell culture bag is provided on one or both of a side of the cell culture bag and a side of the pressing member.

7. The cell culture kit according to claim 6, wherein a sheet member including the slipping surface is interposed between the cell culture bag and the pressing member.

8. The cell culture kit according to claim 6 or 7, wherein the slipping surface is a roughened surface obtained by roughening treatment.

9. The cell culture kit according to claim 8, wherein the roughened surface has surface roughness (Ra) of from 0.01 to 0.70 µm.

10. The cell culture kit according to claim 7, wherein the sheet member is made of a self-lubricating material.
